# EUROPEAN PATENT APPLICATION

(11) **EP 2 422 804 A1**
(43) Date of publication of application: **29.02.2012**
(21) Application number: 11182434.8
(22) Date of filing: 16.06.2005
(51) Int. Cl.: A61K 35/66

(54) **Controlled release formulations of enzymes, microorganisms, and antibodies with mucoadhesive polymers**

(30) Priority: 17.06.2004 US 580105 P
(62) Divisional of application: 05760354.0
(71) Applicant: Amano Enzyme USA., Ltd., Elgin, IL 60123-7872 (US); Amano Enzyme Inc., Nagoya 460-8630 (JP)
(72) Inventor: Jolly, James, F., Elgin, IL 60123-7872 (US)
(74) Representative: Pohlman, Sandra M.

(57) **Abstract**

There is provided a composition comprising at least one mucoadhesive polymer that is capable of forming a hydrogel and at one least water soluble polymer, and one or more enzymes, microorganisms, or antibodies. The formulation forms a hydrogel in aqueous solution that has mucoadhesive properties and that is capable of releasing the enzymes, microorganisms, or antibodies over an extended period of time and/or of entrapping enzymes, microorganisms, or antibodies within the hydrogel that is active for an extended time.

## Description

This application claims the benefit of priority of U.S. Provisional Patent Application No. 60/580,105, filed on June 17, 2004.

### BACKGROUND OF THE INVENTION

The present invention relates generally to the field of enzymes, microorganisms, and antibodies that are formulated into controlled release compositions.

Transmucosal delivery of drugs is a well-established method for delivering drugs, primarily because mucous membranes are relatively permeable. Consequently, the membranes permit the rapid uptake of drugs into systemic circulation. In general, transmucosal drug delivery regimens and/or devices rely upon the mucoadhesive properties of certain polymers that entrain and adhere the drug to the mucous membrane. This conventional technique allows for a drug to be delivered across a mucous membrane, but does not address situations where the drug is intended to stay at or on the mucous surface.

An important subset of therapeutic agents are enzymes, such as, for example, digestive enzymes. A variety of digestive disorders have spawned conventional methodologies for delivering digestive enzymes to the mucous surfaces of the digestive tract. In order to effectively treat such disorders, however, intact enzyme must be at or near a mucous surface and remain at or near the surface long enough to result in efficacious treatment of the disorder. Oral ingestion of an enzyme formulation raises the possibility that the enzyme will be degraded by gastric acid in the stomach. Thus, for example, U.S. Pat. No. 5,302,400 propounds microspheres of digestive enzyme formulations that are coated with a gastric acid-resistant polymer. Other approaches contemplate the immobilization of an enzyme in a polymer carrier that is capable of selectively releasing the enzyme. *See* U.S. Pat. No. 4,975,375. None of these enzyme formulations, however, address the need to have the enzyme remain at the mucous surface or provide extended or controlled release enzyme delivery profiles.

Another problem with the delivery of enzymes by conventional means is the need for the administration of multiple dosages of an enzyme formulation throughout the day. This problem arises because conventional enzyme formulations are swept along the digestive tract and must be replenished to exhibit the intended therapeutic effect. Additionally, conventional digestive enzyme formulations usually must be ingested with each meal. For these reasons, patient compliance is often an issue, particularly with pediatric and geriatric populations.

There remains therefore a need in the art for enzyme formulations that are capable of contacting and remaining at or near mucosal surfaces to provide therapeutic amounts of the enzyme at or near the mucosal surface for an extended period of time.

### SUMMARY OF THE INVENTION

The present invention satisfies these needs and others by providing a composition comprising at least one mucoadhesive polymer that is capable of forming a hydrogel; at least one water-soluble polymer; and a therapeutically effective amount of at least one enzyme. In some embodiments the enzyme is a digestive enzyme, and in other embodiments the enzyme is a therapeutic enzyme.

The invention also provides a composition comprising at least one mucoadhesive polymer that is capable of forming a hydrogel; at least one water-soluble polymer; and at least one strain of a microorganism that can produce a therapeutically effective amount of at least one enzyme. Exemplary microorganisms include bacteria and yeasts.

The invention additionally provides a composition comprising at least one mucoadhesive polymer that is capable of forming a hydrogel; at least one water-soluble polymer; and a therapeutically effective amount of at least one antibody. In some embodiments, the antibody is an antitumor necrosis factor alpha monoclonal antibody.

There is also provided methods of using the compositions of the invention. One embodiment is a method of contacting at least one enzyme with a mucosal surface in a subject in need of the enzyme by administering to the subject a composition of this invention. Another embodiment is a method of aiding digestion in a subject in need of such aid comprising administering the composition to a subject. Yet another embodiment is a method of preventing the digestion of carbohydrates in a subject. Still another embodiment pertains to a method of stimulating the growth of probiotic bacteria in a subject. Another embodiment is a method of contacting at least one enzyme with a mucosal surface in a subject in need of the enzyme by administering to the subject a composition comprising a microorganism that can produce the enzyme according to this invention. Another embodiment provides a method of treating a subject suffering from gastrointestinal or gastrointestinal-related disorders by administering to the subj ect a composition as described herein.

### BRIEF DESCRIPTION OF THE DRAWINGS

FIGURE 1 charts the volume over time for the hydration of Formulations 1-7.

FIGURE 2 shows the time dependence of lactase release (measured as the change in optical density [OD] at 420 nm) from Formulations 1-7.

FIGURE 3 shows the distribution of lactase activity in the supernatant, dispersed hydrogel, and intact hydrogel for each of Formulations 1-7.

FIGURE 4 shows the time dependence of lactase release from Formulations 1-7 that contain pectin instead of polycarbaphil.

FIGURE 5 shows the time dependence of lactase release from Formulations 1-7 that contain gum Arabic instead of polycarbophil.

FIGURE 6 shows the distribution of lipase activity in the supernatant, dispersed hydrogel, and intact hydrogel for each of Formulations 1-7.

FIGURE 7 shows the distribution of protease activity in the supernatant, dispersed hydrogel, and intact hydrogel for each of Formulations 1-7.

FIGURE 8 shows the mucoadhesive character (work of adhesion) for each of formulations 1-7.

FIGURE 9 shows the mucoadhesive character (work of adhesion) for three formulations based on formulation 3 containing polycarbophil, pectin, and gum Arabic, respectively.

### DETAILED DESCRIPTION

Unless defined otherwise, the terms used herein are intended to have their ordinary meaning in the art. Unless otherwise specified, "a," "an" or "the" designates one or more, and words used in the singular also indicate the plural.

A "pharmaceutical composition" refers to a mixture of one or more of the enzymes, microorganisms, or antibodies described herein, or physiologically acceptable salts thereof, with other chemical components, such as physiologically acceptable carriers and excipients as described below. The purpose of a pharmaceutical composition is to facilitate administration of an enzyme, microorganism, or antibody to an organism.

The present invention provides a composition comprising a mucoadhesive polymer that is capable of forming a hydrogel; a water-soluble polymer, and a therapeutically effective amount of at least one enzyme, microorganism, or antibody.

In one embodiments, the composition is a pharmaceutical composition.

The present inventor found that a hydrogel-forming composition can be made that entrains an enzyme, microorganism, or antibody, adheres to a mucosal surface, and also retains biological activity, either within the hydrogel or by releasing the enzyme, microorganism, or antibody. The inventor discovered that the composition is able to entrain one or more of these components and maintain them in contact with or near a mucosal surface. The composition can be formulated to retain the enzyme, microorganism, or antibody at or near the mucosal surface for an extended period. Alternatively, it can be formulated to release the enzyme, microorganism, or antibody at or near the mucosal surface. Thus, for example, the composition may adhere to the mucosal surface and permit ingress of enzyme, microorganism, or antibody substrate, or it may release enzyme, microorganism, or antibody at or near the mucosal surface, or both, in a controlled manner.

In one embodiment, the composition may comprise any enzyme or mixture of enzymes. A suitable subset of enzymes is digestive enzymes, which are implicated in the treatment, prophylaxis, prevention, or operation of digestive disorders or processes. In one embodiment, the digestive enzyme is a protein reducing enzyme, including, for example, proteases and peptidases such as exoprotease, endoprotease, and acid stable proteases; bromelain; papain; and actinidin. In another embodiment, the enzyme is a fiber reducing enzyme. Exemplary fiber reducing enzymes include endocellulase, glucanase, hemicellulase, and pectinase. In other embodiments, the enzyme is a fat and/or oil reducing enzyme, such as, for example, a lipase or lipase blend. In yet other embodiments, the enzyme is a starch reducing enzyme, including but not limited to alpha amylase, beta amylase, and amyloglucosidase. In a further embodiment, the enzyme is a sugar and/or dairy enzyme. Exemplary enzymes in this category include lactase, invertase, maltase, xylanase, alpha galactosidase, and mannanase.

Other enzymes useful in the composition include, for example, cellulase. Still others include transglucosidase, fructofuranosidase, levansucrase, which converts the carbohydrate breakdown products into oligosugars, which are not absorbed by the intestines. Such compositions are useful, for example, in the treatment of pre-diabetics, for weight management, and in supporting the growth of beneficial intestinal bacteria.

The invention also includes compositions comprising the enzyme bilirubin oxidase, which breaks bilirubin into a secretable form, and are useful, for example, in treating jaundice.

The invention also includes compositions comprising the enzyme nattokinase, a fibrinolytic enzyme.

The invention also includes compositions comprising α-galactosidase, phytase, oxylate oxidase, β-glycosidase, collegenase, mutanase and dextranase.

The mucoadhesive polymer is selected from mucoadhesive polymers that are capable of forming hydrogels. In accordance with the accepted meaning in the art, a hydrogel is a colloidal gel in which water is the dispersion medium. Thus, a mucoadhesive polymer capable of forming a hydrogel will swell in the presence of water. In the context of the present invention, it is believed that the mucoadhesive polymer serves to maintain the composition in contact with or near a mucous surface while the ability of the polymer to form a hydrogel is believed to entrap and thus immobilize the one or more enzymes of the composition.

Within the general guidelines prescribed above, any mucoadhesive polymer that can form a hydrogel is contemplated for use in the composition. Many such polymers are known in the art. Preferred mucoadhesive polymers include but are not limited to carbopols, N-isopropylacrylamide, polyvinyl alcohol/polyvinyl pyrrolidone, dextran, hydroxyethylmethacrylate/methacrylic acid, polyvinyl alcohol, polyacrylamide, polyethylene glycol/poly lactic acid, carboxymethyl chitosan and collagen. Exemplary polymers in this regard are polycarbophil and other acrylate/methacrylate polymers.

Further exemplary polymers in this regard include anionic polymers based on methacrylic acid esters, such as the Eudragit^{®} (Degussa) family of polymers, which form hydrogels that dissolve and can thereby release an entrained enzyme within prescribed pH ranges, generally between about pH 5.5 to about pH 7.5. For example, such a polymer that dissolves in the pH range from about 5.5 to about 6.0 is useful for targeting the duodenum. A polymer that dissolves at increasing pH generally targets lower sections of the intestine, where near the colon, for example, the pH is about 6.5 to about 7.0. In this manner, the enzyme that is entrained in the hydrogel can avoid the decomposition and/or release in the stomach, where the pH is much lower. Finally, combinations of two or more any of the foregoing mucoadhesive polymers are also contemplated.

The inventive composition also comprises a water-soluble polymer. This polymer may or may not form a hydrogel to some extent when hydrated, but is nonetheless able to dissolve in aqueous media. In the context of the invention, it is believed that this polymer acts to hydrolyze the hydrogel formed by the mucoadhesive polymer. Many water-soluble polymers are known in the art. Suitable polymers in this regard include but are not limited to polyols and polycarbohydrates. Exemplary water-soluble polymers include hydroxylated celluloses, such as, for example, hydroxypropylmethyl cellulose and hydroxymethyl cellulose. Other suitable water-soluble polymers include chitosan, polyethylene glycol, polsorbate 80, starch acetate and polylactic acid. Combinations of two or more water-soluble polymers are also contemplated.

It is believed that the mixture of mucoadhesive and water-soluble polymers gives rise to advantageous properties of the composition. The relative concentrations and identities of these polymers may be adjusted to tailor or optimize a number of properties of the composition, some of which may be competing. These properties include, for example, the mucoadhesion of the composition, the distribution and amount of enzyme entrapped within the composition, the amount of enzyme that is released from the composition, and the rate at which the enzyme is released.

Depending on the enzyme(s), microorganisms, and antibodies and the conditions being treated or prevented, it may be desirable to provide a composition that retains the enzyme for a long period of time. For example, lactase is normally found in the intestine wall, and thus it may be desired to retain lactase within the composition at or near the mucosal surface of the intestine wall. On the other hand, it may be desirable to provide some release of the enzyme, microorganism, or antibody. For example, some protease is released into intestinal fluid. Thus, it may be desired to release some protease from the composition into intestinal fluid. Those skilled in the art can readily determine desired retention/release profiles, and can choose polymers and adjust their relative concentrations to achieve desired profiles.

The invention also provides a composition comprising the polymer mixture as described above and at least one microorganism that is capable of producing a therapeutically effective amount of at least one enzyme. In this context, the composition engenders not only the controlled release of the enzyme, but also a sustained release of the enzyme as a consequence of the microorganism continually producing the enzyme. Many microorganisms are known in the art to exhibit this function. Exemplary microorganisms include but are not limited to bacteria and fungi. Some bacteria that are useful in this regard are bifidobacteria and lactobacilli, such as, for example, *Bifidobacterium lactis* and other *Bifidobacterium* species, *L*. *acidophilus, L. rhamnosus,* and *L. plantarum.* Examples of fungi include yeasts, such as, for example, *Saccharomyces boulardii.* Pairings of microorganisms and the enzyme(s) that they produce are well known in the art. For example, *bacillus coagulans* strains are known to be sources of enzymes such as endonucleases (U.S. Pat. No. 5,200,336), amylase (U.S. Pat. No. 4,980,180), lactase (U.S. Pat. No. 4,323,651), and cyclo-malto-dextrin glucano-transferase (U.S. Pat. No. 5,102,800). Additionally, various *lactobacillus* cultures that are known in the art produce lactase (U.S. Pat. No. 6,461,607 and references cited therein).

Other embodiments described below relate to the methods of using the present enzyme compositions to stimulate the growth of such microorganisms, which are recognized themselves as being therapeutically beneficial or else capable of providing beneficial enzymes. In this context, therefore, the embodiments are complementary in that the present compositions can be adapted to stimulate the growth of existing and beneficial microorganisms, or provide the microorganisms themselves. In both embodiments, a subject in need receives benefits from both methods.

Other embodiments of the invention provide a composition comprising the polymer mixture as described above and a therapeutic amount of at least one antibody. The identity of the antibody is not essential and therefore can be selected from antibodies that are known to the person of skill in the art. In some embodiments described below, the antibodies are selected according to their known prophylactic properties against gastrointestinal or gastrointestinal-related disorders. Thus, for example, antibodies useful in these embodiments include but are not limited to antitumor necrosis factor alpha monoclonal antibodies, anti-interleukin-12 antibodies, and anti-rotaviral antibodies. A specific example of an antitumor necrosis factor alpha monoclonal antibody is infliximab.

Although the concentrations of the polymers can vary considerably, the amount of mucoadhesive polymers or mixtures thereof generally ranges from about 1 to about 99%, from about 10 to about 75%, from about 15 to about 67%, from about 20 to about 60%, and from about 25 to about 50% (w/w based on the weight of total polymer). The amount of water-soluble polymers or mixtures thereof can range from about 1 to about 99%, from about 20 to about 90%, from about 30 to about 85%, from about 40 to about 80%, and from about 55 to about 75% (w/w based on the weight of total polymer).

Illustrative formulations of the invention comprise hydroxypropylmethylcellulose as the water-soluble polymer and carbopol as the mucoadhesive polymer. Exemplary concentration ranges ofmucoadhesive polymer in this context include from about 1 to about 50%, from about 10 to about 45%, from about 15 to about 40%, and from about 20 to about 35% (w/w based on the weight of total polymer). Exemplary concentration ranges of water-soluble polymer include from about 45 to about 99%, from about 50 to about 90%, from about 55 to about 85%, and from about 60 to about 80% (w/w based on the weight of total polymer). See also Formulations 2-6 of the Examples.

The composition may further comprise other components, such as one or more pharmaceutically acceptable binding agents, lubricating agents, suspending agents, sweeteners, flavoring agents, preservatives, buffers, wetting agents, disintegrants, effervescent agents, and other excipients. Such excipients are known in the art. In one embodiment, the composition of the present invention is blended with at least one pharmaceutically acceptable excipient, diluted by an excipient or enclosed within a carrier that can be in the form of a capsule, sachet, tablet, buccal, lozenge, paper, or other container. When the excipient serves as a diluent, it may be a solid, semi-solid, or liquid material which acts as a vehicle, carrier, or medium for the balance of the composition. Thus, the composition can be formulated into tablets, pills, powders, elixirs, suspensions, emulsions, syrups, capsules (such as, for example, soft and hard gelatin capsules), suppositories, lozenges, buccal dosage forms, sterile injectable solutions, and sterile packaged powders.

Examples of binding agents are various celluloses and cross-linked polyvinylpyrrolidone, microcrystalline cellulose, such as Avicel PH101 and Avicel PH102, microcrystalline cellulose, silicidized microcrystalline cellulose (SMCC), and mannitol.

Suitable lubricants, including agents that act on the flowability of the powder formulation to be compressed, are colloidal silicon dioxide, such as Aerosil 200; talc, stearic acid, magnesium stearate, calcium stearate, and silica gel.

Examples of sweeteners are any natural or artificial sweetener, such as sucrose, xylitol, sodium saccharin, cyclamate, aspartame, and acesulfame and salts thereof. Examples of flavoring agents are Magnasweet (trademark of MAFCO), bubble gum flavor, fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, dibasic calcium phosphate, saccharides, and/or mixtures of any of the foregoing. Examples of diluents include microcrystalline cellulose, such as Avicel PH101 and Avicel PH102; dibasic calcium phosphate such as Emcompress; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the acid component of the effervescent couple may be present.

In one embodiment, the composition comprises the following components in addition to the mucoadhesive polymer, water-soluble polymer, and enzyme, microorganism, or antibody: one or more pharmaceutically acceptable binders and/or lubricants.

In some embodiments of the invention, the composition is made into the form of dosage units for oral administration. For example, the polymers and one or more enzymes, microorganisms, or antibodies may be mixed with a solid, pulverant carrier such as, for example, sorbitol, mannitol, starch, amylopectin, cellulose derivatives or gelatin, as well as with an antifriction agent such as, for example, magnesium stearate, calcium stearate, and polyethylene glycol waxes. The mixture is then pressed into tablets. If coated tablets are desired, the above prepared core may be coated, such as with a concentrated solution of sugar, which may contain gum arabic, gelatin, talc, titanium dioxide, or with a lacquer dissolved in volatile organic solvent or mixture of solvents. To this coating, various dyes may be added in order to distinguish among tablets with different active compounds or with different amounts of the active compound present.

Soft capsules also may be prepared, such as capsules which contain a mixture of the polymers and one or more enzymes, microorganisms, or antibodies with vegetable oil or non-aqueous, water miscible materials such as, for example, polyethylene glycol and the like. Hard capsules may contain granules of the composition in combination with a solid, pulverant carrier, such as, for example, sorbitol, mannitol, potato starch, corn starch, amylopectin, cellulose derivatives, or gelatin.

Dosage units for rectal administration may be prepared in the form of suppositories which may contain the enzyme and polymers mixture with a neutral fat base, or they may be prepared in the form of gelatin-rectal capsules which contain the formulation in a mixture with a vegetable oil or paraffin oil.

The invention contemplates, in one embodiment, granulated forms of the compositions described herein. Granulated forms are useful for preparing tablets for oral use, and they are typically prepared in the following manner, although other techniques well known in the art may be employed. The solid substances are gently ground or sieved to a desired particle size, and the resulting mass is gently pressed through a stainless steel sieve having a desired size. The layers of the mixture are then dried in controlled drying units for a determined length of time to achieve a desired particle size and consistency. The granules of the dried mixture are gently sieved to remove any powder. To this mixture, disintegrating, anti-friction, and anti-adhesive agents are added. Finally, the mixture is pressed into tablets using a machine with the appropriate punches and dies to obtain the desired tablet size. The operating parameters of the machine may be selected by the skilled artisan.

Preparation of lozenge and buccal dosage forms can be effected by methods known to one of ordinary skill in the art.

Compositions of the present invention, when used under the conditions prescribed herein, may be formulated to release the enzyme, microorganism, or antibody over an extended period of time, such as, for example, between about two to about sixteen hours. In some embodiments, the enzyme, microorganism, or antibody is released between about three to about twelve hours. In yet other embodiments, the enzyme, microorganism, or antibody is released between about four to about eight hours. The composition may be formulated so as not to release all of the enzyme, microorganism, or antibody. Enzyme, microorganism, or antibody remaining in the composition exhibits biological activity for at least 24 hours. Thus, for example, the invention permits the release of an amount of enzyme and also the retention of an amount of enzyme at or near the mucosal surface.

In some embodiments, compositions described herein also may be formulated in the form of nanoparticles. The nanoparticulate formulations feature an effective average particle size of less than about 2000 nm. The term "effective average particle size of less than about 2000 nm" means that at least 50% of the formulation particles have a weight average particle size of less than about 2000 nm when measured by light scattering or other conventional techniques. In some embodiments,, at least 70% of the formulation particles have an average particle size of less than about 2000 nm. In other embodiments, at least 90% of the formulation particles have an average particle size of less than about 2000 nm. In still other embodiments, at least about 95% of the formulation particles have a weight average particle size of less than about 2000 nm.

Some embodiments provide for the nanoparticulate formulation to have an effective average particle size of less than about 1000 nm. In other embodiments, the effective average particle size is less than about 500 nm.

The nanoparticulate formulations as contemplated herein can be made by conventional formulation techniques known to those who are skilled in the art. Thus, for example, a composition described herein can be dissolved in a minimum volume of water.

The composition may be dissolved in a variety of ways. The methods include but are not limited to heating, sonicating, high shearing, or high stirring.

A countersolvent, typically an alcohol, is then introduced to the aqueous solution of the composition. Nanoparticles of the composition are then precipitated. The nanoparticulate formulation can be isolated and dried for further use. Variations and adaptations of this general method are described, for example, in U.S. Pat. No. 6,824,791.

Examples of an alcohol countersolvent are: methanol (methyl alcohol), ethanol, (ethyl alcohol), 1-propanol (n-propyl alcohol), 2-propanol (isopropyl alcohol), 1-butanol (n-butyl alcohol), 2-butanol (sec-butyl alcohol), 2-methyl-1-propanol (isobutyl alcohol), 2-methyl-2-propanol (t-butyl alcohol), I-pentanol (n-pentyl alcohol), 3-methyl-l-butanol (isopentyl alcohol), 2,2-dimethyl-1-propanol (neopentyl alcohol), cyclopentanol (cyclopentyl alcohol), 1-hexanol (n-hexanol), cyclohexanol (cyclohexyl alcohol), 1-heptanol (n-heptyl alcohol), 1-octanol (n-octyl alcohol), 1-nonanol (n-nonyl alcohol), 1-decanol (n-decyl alcohol), 2-propen-1-ol (allyl alcohol), phenylmethanol (benzyl alcohol), diphenylmethanol (diphenylcarbinol), triphenylmethanol (triphenylcarbinol), glycerin, phenol, 2-methoxyethanol, 2-ethoxyethanol, 3-ethoxy-1,2-propanediol, Di(ethylene glycol) methyl ether, 1,2-propanediol, 1,3-propanediol, 1,3-butanediol, 2,3-butanediol, 1,4-butanediol, 1,2-pentanediol, 1,3-pentanediol, 1,4-pentanediol, 1,5-pentanediol, 2,3-pentanediol, 2,4-pentanediol, 2,5-pentanediol, 3,4-, pentanediol, and 3,5-pentanediol. The countersolvent may also be a mixture of alcohols selected from the aforementioned group.

Particle size can be controlled by varying the relative concentrations of composition components, polymer concentrations, and the solvent/countersolvent volumetric ratios. The latter can vary from about 1:40 and about 1:1,000,000, and typically varies from about 1:50 to about 1:200.

While not wishing to be bound by any particular theory, the inventor believes that certain advantages of the invention flow from the presence of a combination of polymers with different properties. Thus, it is believed that a composition comprising one or more polymers exhibiting only mucoadhesive properties would disintegrate relatively quickly in the methods prescribed herein. In that scenario, the one or more enzymes, microorganisms, or antibodies would be eliminated by the patient, and in any event would not be entrapped within a hydrogel, thereby depriving a subject of the controlled release therapeutic profile that is possible with the present invention. On the other hand, a composition comprising only mucoadhesive polymers that form hydrogels may immobilize enzymes, microorganisms, or antibodies to such an extent that these active agents are prevented from contacting relevant substrates. The present invention therefore provides at least one water-soluble polymer in the composition to balance this property. In this regard, it is believed that the water-soluble polymer facilitates egress of enzyme, microorganism, or antibody and ingress of substrate into the hydrogel formed by the composition.

The invention also provides methods of using the composition. In one embodiment, there is provided a method of contacting a therapeutically effective amount of at least one enzyme, microorganism, or antibody with a mucosal surface in a subj ect in need of that enzyme, microorganism, or antibody by administering to the subject a composition of the invention. The term "therapeutically effective amount" as used herein refers to that amount of the enzyme, microorganism, or antibody being administered which will relieve, treat, or prevent to some extent one or more of the disorders or symptoms of the disorders being treated.

A therapeutically effective amount of an enzyme varies considerably depending on the enzyme of interest and the activity of that enzyme. Generally, therapeutically effective amounts of the enzymes can be readily determined by those who are skilled in the art. Illustrative daily doses and dosing regimens for a number of enzymes (dietary supplement (DS), AmanoEnzyme USA) is given in the following table:

| **Enzyme** | **Daily Minimum** | **Daily Maximum** | **Dose Regimen (number tablets ×** |
|---|---|---|---|
| | **Dose (g)** | **Dose (g)** | **dose/tablet (g))** |
| Protease DS | 0.105 | 0.31 | 3 × 0.1 |
| Cellulase S-DS | 0.18 | 0.36 | 2 × 0.1 |
| Lipase DS | 0.045 | 0.09 | 1 × 0.05 |
| Amylase DS | 0.3 | 0.6 | 3 × 0.1 |
| α-galactosidase DS | 0.15 | 0.3 | 2 × 0.1 |
| Lactase DS | 0.3 | 0.6 | 3 × 0.1 |

The administration can be achieved through any of the routes as mentioned above. In embodiments where the enzyme, microorganism, or antibody is to be contacted with mucous surfaces in the small intestine, for example, the administering may occur by oral administration (including oral ingestion) of the composition. Other routes include directly contacting the composition to the mucous surface of interest.

Exemplary mucous surfaces include but are not limited to corneal, conjunctival, nasal, buccal, sublingual, pulmonary, stomachic, intestinal, uteral, bladder, rectal, and vaginal mucosa. Compositions suitable for contacting enzymes, microorganisms, or antibodies with specific mucous surfaces can be made by using compositions that form hydrogels at a pH within the range found at the mucous surface. Thus, for example, a composition for contact with the stomach can be formulated to form a hydrogel at a pH of about 4, while a composition for contact with the intestines can be formulated to form a hydrogel at a pH of about 6-8. Additionally or alternatively, the composition may be contacted by direct application to the mucosal surface of interest, such as for example, to corneal, conjunctival, nasal, buccal, sublingual, uteral, bladder, rectal, and vaginal mucosal surfaces.

In a particular embodiment, the invention provides a method of aiding digestion in a subject, comprising administering to the subject a composition of this invention comprising one or more digestive enzymes. Exemplary digestive enzymes are set forth above. The person of skill in the art can select one or more enzymes to target a specific digestive need, disorder, or condition. Thus, for example, compositions comprising lactase would be suitable for use in the method where a subject exhibits lactose intolerance.

In another embodiment, the invention provides a method of preventing the digestion of carbohydrates in a subject, comprising administering to the subject a composition of this invention comprising, for example, a transglucosidase, levansucrase, biodiastase, or a mixture thereof. As discussed above, such a method is useful in treating pre-diabetic subjects and for weight control.

In another embodiment, the invention provides a therapeutic method comprising administering to the subject a composition of this invention comprising nattokinase.

In another embodiment, there is provided a method of stimulating the growth ofprobiotic bacteria in a subject comprising administering to a subject a composition of this invention, Suitable enzymes that can be used to stimulate the bacteria in this method include but are not limited to a transglucosidase, levansucrase, biodiastase, or a mixture thereof. Probiotic bacteria generally favorably alter the intestinal microflora balance, inhibit the growth of harmful bacteria, promote good digestion, boost immune function, and increase resistance to infection. Exemplary probiotic bacteria include but are not limited to bifidobacteria such as *Bifidobacterium lactis,* which boosts immune function; and lactobacilli such as *L. acidophilus, L. rhamnosus,* and *L. plantarum,* which serve as protective intestinal bacteria. This embodiment therefore complements compositions and uses described above that provide for the delivery of the microorganisms themselves, such as bacteria described herein, to a subject in need.

Another embodiment, as noted above, provides a method of contacting at least one enzyme with a mucosal surface in a subject in need of the enzyme. In this embodiment, the method can be achieved by administering to the subj ect a composition comprising the polymer mixture as described herein together with a strain of a microorganism capable of producing the enzyme. The microorganism thus produces a therapeutically effective amount of the enzyme that is to be contacted with the mucosal surface.

Another embodiment provides for treating a subject suffering from a gastrointestinal or gastrointestinal-related disorder. The method entails administering to the subject a composition comprising a composition comprising an antibody as described herein. While the identity of the antibody is not crucial, it should be selected according to known therapeutic regimens used for treating the disorders. Thus, for example, one embodiment relates to treatment of Crohn's disease. It is known in the art that infliximab, an antitumor necrosis factor alpha monoclonal antibody, is useful in the treatment of Crohn's disease. Other antibody-disorder pairings can be similarly identified by reference to conventional treatment regimens. This embodiment thus exploits one advantage of the inventive composition by placing and maintaining the therapeutic antibody at the site affected by the disorder.

Gastrointestinal and gastrointestinal-related disorders are intended to encompass those disorders that occur within or affect the gastrointestinal tract, or result from or otherwise occur in conjunction with other disorders. Specific examples of disorders than can be treated in this context are Crohn's disease, ulcerative colitis, lymphomas, and a viral gastroenteritis, such as rotovirus. The antibody compositions are also useful in the treatment of subjects that suffer from organ rejection after intestinal transplantation.

In the foregoing methods, the subj ect can be any organism with a mucosal surface. In accordance with one aspect of the invention, the subj ect is a mammal. In accordance with another aspect of the invention, the subject is a human being.

The following examples are intended to further describe the invention by way of illustration, and thus should not be construed as limiting the scope of the invention in any way. All publicly available documents, including patents, are expressly incorporated as if fully set forth herein.

### General

### Materials

Carbopol 934 (polycarbophil) was obtained from Noveon, Inc. (Cleveland OH); hydroxypropylmethyl cellulose (HPMC, Methocel K100M) was obtained from Dow Chemical Co. (Midland, MI); mannitol, pectin and gum arabic were obtained from Sigma Chemical Co.; and magnesium stearate (FDA grade) was obtained from Tabletpress.net (Athens, OH). All enzymes were supplied by Amana Enzyme USA Co., Ltd.

### Equipment

Tablet formulations were prepared using a Benchtop Model Single-Punch Tablet Press (TDP) from Tabletpress.net (Athens, OH); software for texture analysis was TA.XTplus from Texture Technologies Corp. (Scarsdale, NY), and formulations were blended using an Inversina Power Blender from Laval Lab (Quebec, Canada).

### Formulations

Each tablet formulation indicated in Table 1 below was prepared by mixing 1.5 mg mannitol as a binding agent, 0.3 mg magnesium stearate as a lubricant 100 mg of enzyme and 150 mg of total polymer per tablet in the blender at speed 5 for 2 hours. The individual mixtures were then pressed manually with the tablet press to yield tablets (8x3 mm). The polymer component of each formulation varied according to the concentration of carbopol 934 and hydroxypropylmethylcellulose (HPMC).

**Table 1**

| **Formulation** | **HPMC** | | **Carbopol 934** | |
|---|---|---|---|---|
| | grams | wt% | grams | wt% |
| **1** | 1.50 | 100 | 0.00 | 0 |
| **2** | 1.25 | 80 | 0.25 | 20 |
| **3** | 1.00 | 67 | 0.50 | 33 |
| **4** | 0.75 | 50 | 0.75 | 50 |
| **5** | 0.50 | 33 | 1.00 | 67 |
| **6** | 0.25 | 25 | 1.25 | 75 |
| **7** | 0.00 | 0 | 1.50 | 100 |

### Example 1: Tablet Hydration

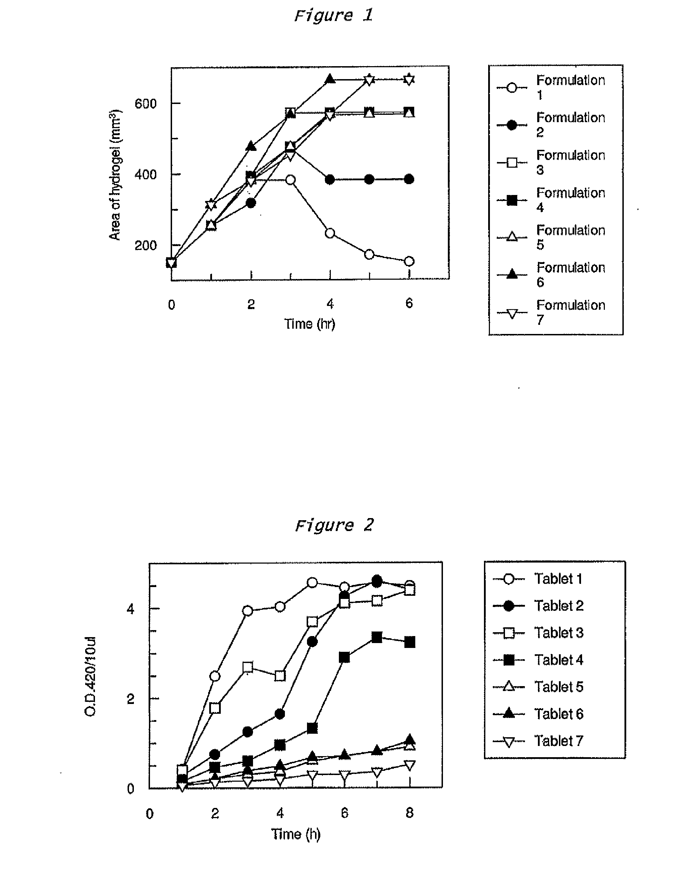

Each tablet was placed in a plastic weight boat with 20 ml of pre-warmed PBS buffer (37°C; pH 7.2) and floated on the surface of a 37°C water bath. At the hourly intervals indicated in FIGURE 1, the buffer was removed and the size of the tablet was measured. After measurement, 20 ml of pre-warmed PBS buffer was added and the weight boat was again placed on the surface of the water bath.

As shown in FIGURE 1, Formulation 1 swelled initially and then gradually dissolved. Formulations 2-7 show an increase in volume with formulations containing the most polycarbophil (tablets 6 and 7) swelling up to 7 times the original volume of the tablet. The tablets containing polycarbophil form hydrogel structures that are stable for more than 24 hours.

### Example 2: Lactase Release

Formulations and tablets corresponding to Formulations 1-7 were prepared with 100 mg of Lactase DS per tablet. One tablet of each formulation was placed into a 50 ml conical tube with 10 ml of PBS buffer (pH 7.2) and incubated at 37.5°C. At the hourly time intervals indicated in FIGURE 2,10 *µ*l was removed and assayed for lactase activity using the FCCIV assay procedure for lactase (Institute of Medicine, Food Chemicals Codex, Fourth Edition, National Academy Press, Washington, D.C., 1996).

FIGURE 2 shows that the release of lactase from Formulation 1 was rapid and complete within 2 hours. Release of lactase from formulations 2-7 decreased with increasing concentrations of polycarbophil. The time that lactase release from formulations 2-4 was observed increased to 6 hours.

### Example 3: Lactase Distribution

Lactase tablets with Formulation 1-7 were placed one tablet each into 50 ml conical tubes with 5 ml of PBS buffer. The tubes were incubated for 16 hours at 37.5°C. The supernatant was removed from each tube and assayed for lactase activity. The remaining hydrogel was washed with 3 x 5 ml of PBS buffer and then disrupted in 5 ml of PBS buffer and the suspension was assayed for lactase. Since the tablet with Formulation 1 dissolved completely, the lactase activity in the tube with Formulation 1 was taken to be 100% of the lactase activity in the tablets.

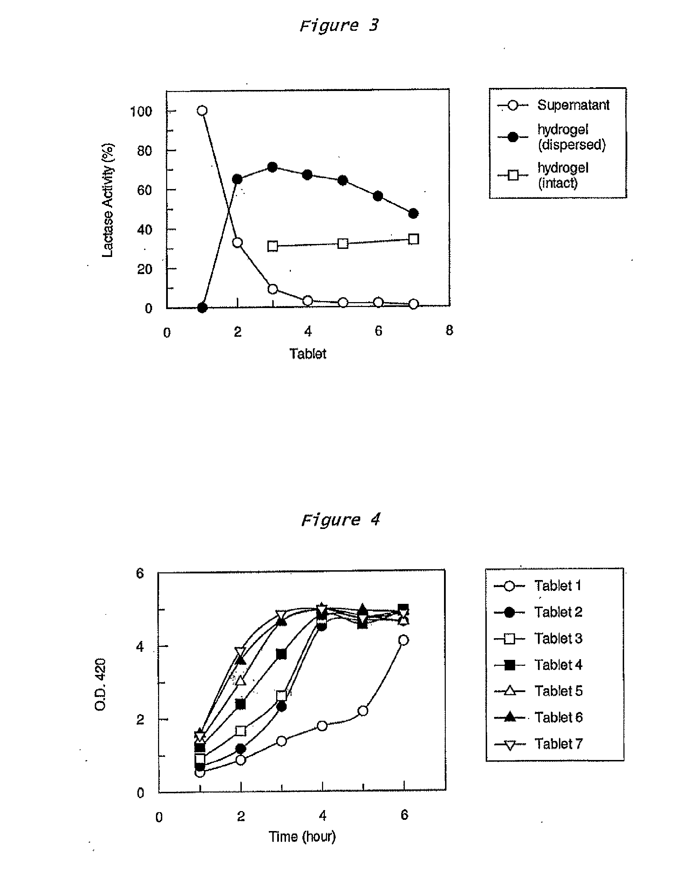

The distributions of lactase activity, either released to the buffer or retained in the hydrogel, is presented in FIGURE 3. It is seen that the amount of lactase released into the buffer decreased significantly with the amount of polycarbophil in the tablet (as noted above, the amount of lactase released from tablet 1 is taken as 100%). Conversely, the amount of lactase immobilized within the hydrogel is relatively constant in the hydro gels resulting from tablets 2-7. Since the concentration of immobilized lactase was virtually invariable, one only need to make a desired formulation by selecting the desired mucoadhesive properties and the amount of lactase that is intended to be released from the formulation. The other Examples herein suggest that tablets of formulations 2 and 3 are optimized with respect to both of these remaining properties.

### Comparative Example 4: Lactase Release (Pectin Tablets)

Tablets with Formulations 1-7 containing 100 mg of Amano Lactase but Pectin instead of carbopol were used in the lactase release experiment following the procedure as described in Example 9. Pectin does not form a hydrogel.

FIGURE 4 shows that when polycarbophil is replaced by a mucoadhesive polymer that does not form a hydrogel (pectin), lactase is released from tablets 2-7 rapidly, and is released from tablet 1 less rapidly (tablet 1 does not have any pectin).

### Comparative Example 5: Lactase Release (Gum Arabic Tablets)

Tablets with formulation 1-7 containing 100 mg of Amano Lactase and Gum Arabic instead of Carbopol were used in the lactase release experiment following the procedure as described in Example 9. Gum Arabic does not form a hydrogel.

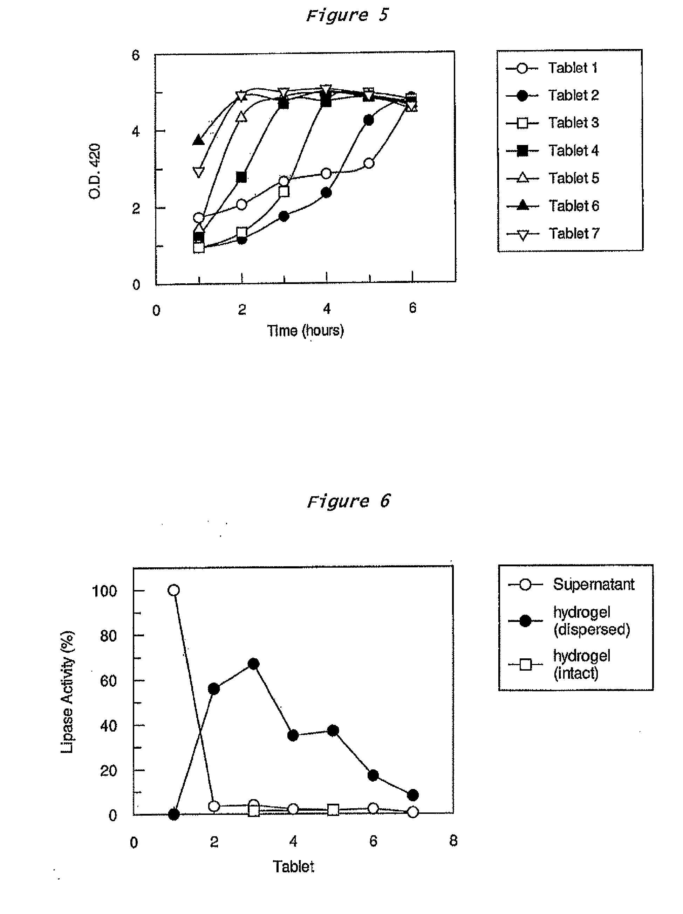

FIGURE 5 indicates that when polycarbophil is replaced by another mucoadhesive polymer that does not form a hydrogel (gum Arabic), lactase is released from tablets 2-7 rapidly, and is released from tablet 1 less rapidly (tablet 1 does not have any gum Arabic).

### Example 6:

a. Lipase Distribution

Tablets with Formulations 1-7 containing 100 mg of Amano Lipase DS were used for the enzyme distribution test following the procedure as described in Example 3. Lipase activity was assayed by Lipase Kit S, Dainippon Pharmaceutical Co., Osaka, Japan.

b. Protease Distribution

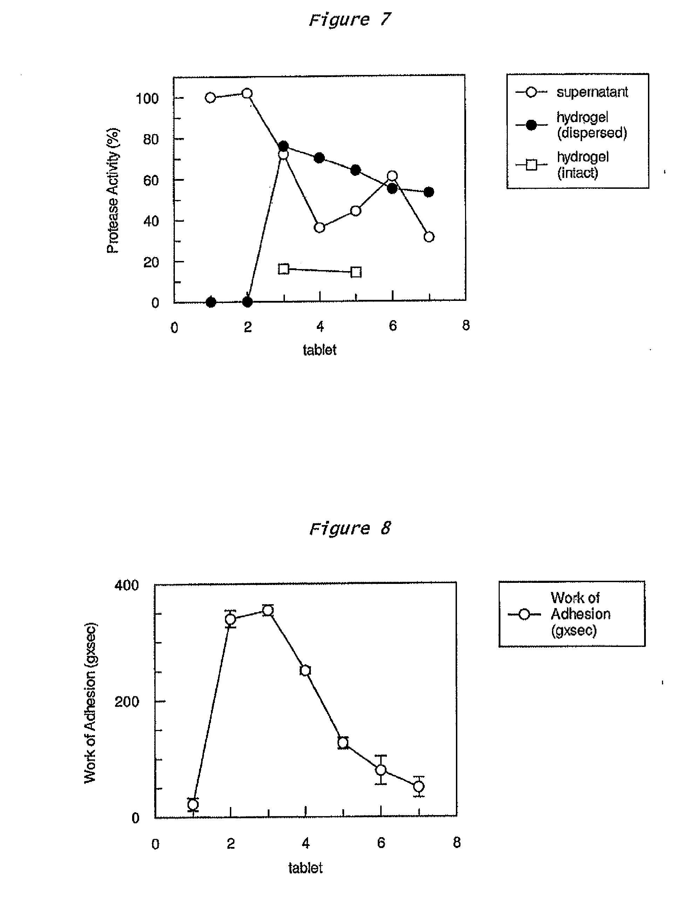

Tablets with Formulations 1-7 containing 100 mg of Amana Protease DS were used for an enzyme distribution test following the procedure as described in Example 3. Protease was assayed by the Fungal acid protease FCCIV method. When lactase was replaced with either lipase or protease in tablets containing polycarbophil, the resulting enzyme distribution between the buffer and hydrogel are similar to the results found with lactase (FIGS. 6 and 7). (Note that in both cases, the enzyme activity assays are less sensitive for enzyme in the intact hydrogel.)

### Example 7: Mucoadhesion Studies

a. Tablets of Formulations 1-7 were taped with two sided tape to the bottom of the probe of a Texture Analysis Instrument (TA.XT plus), Texture Technologies Corp., Scarsdale, NY, and contacted with a solution of 2% porcine stomach mucin present on the surface of a microscope slide. The work of adhesion required to remove the tablet from the mucin surface was determined with the provided Texture Exponent 32 software.

The mucoadhesion of tablet 1 (with no mucoadhesive polymer) was very low while the mucoadhesion of tablets 2 and 3 were at the maximum level obtained (FIG. 8). The mucoadhesion of tablets 4-7 decreased rapidly with further increasing amounts of mucoadhesive polymer.

The results indicate that mucoadhesion requires the presence of polycarbophil (absent in tablet 1) but also indicates that the concentration of polycarbophil is important since at higher levels of polycarbophil, a decrease in mucoadhesion is observed. This may be due to the hydrogel-forming properties of the mucoadhesive polymer, which may dilute to some extent its mucoadhesive properties.

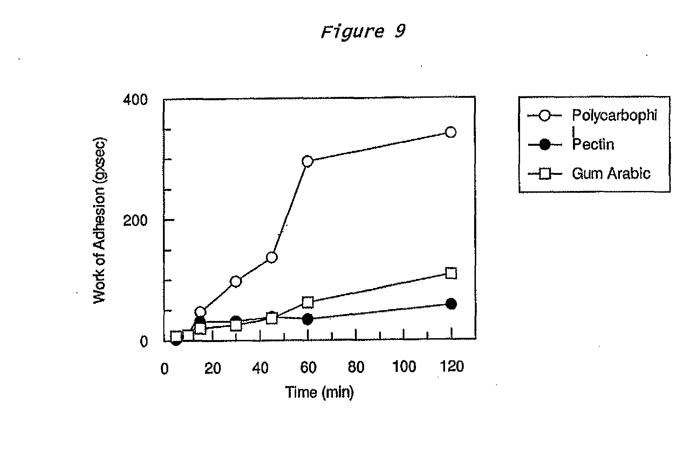

b. A trio of similar experiments were performed on three tablets, respectively, that employed formulation 3 and two variations thereof: (i) polycarbophil (original formulation), (ii) polycarbophil replaced by pectin, and (iii) polycarbophil replaced by gum Arabic, respectively. A time course of mucoadhesion indicated that the maximum level of mucoadhesion was obtained after 60 minutes (FIG. 9). These results indicate that, with a high level of mucoadhesion, hydration of the tablet initiates the formation of a hydrogel. By contrast, the mucoadhesion of tablets formulated with pectin or gum Arabic were not strongly mucoadhesive, probably because they do not form hydrogel structures.
In view of the foregoing, it will be appreciated that the invention described herein *inter alia* relates to the following items:
1. A composition comprising:
   (a) at least one mucoadhesive polymer that is capable of forming a hydrogel;
   (b) at least one water-soluble polymer; and
   (c) a therapeutically effective amount of at least one enzyme.
2. The composition according to item 1, wherein the composition comprises a digestive enzyme.
3. The composition according to item 2, wherein the digestive enzyme is selected from the group consisting of exoprotease, endoprotease, and acid stable proteases, bromelain, papain, and actinidin, endocellulase, glucanase, hemicellulase, cellulase, pectinase, lipase, lipase blends, alpha amylase, beta amylase, amyloglucosidase, lactase, invertase, maltase, xylanase, alpha galactosidase, mannanase, and combinations thereof.
4. The composition according to item 3, wherein the digestive enzyme is selected from the group consisting of lactase, lipase, protease, amylase, and combinations thereof.
5. The composition according to item 4, wherein the composition comprises lactase.
6. The composition according to item 1, wherein the composition comprises a therapeutic enzyme.
7. The composition according to item 6, wherein the therapeutic enzyme is selected from the group consisting of transglucosidase, bilirubin oxidase, β-glycosidase, oxalate oxidase, phytase, collagenase, nattoldnase, fructofuranosidase, levansucrase, and combinations thereof.
8. The composition according to item 1, wherein the mucoadhesive polymer is selected from the group consisting of polyacrylate and/or polymethacrylate polymers.
9. The composition according to item 8, wherein the polyacrylate-methacrylate polymers are selected from carbopols and anionic polymers of methacrylic acid esters.
10. The composition according to item 1, wherein the water-soluble polymer is selected from the group consisting of cellulose, cellulose derivatives, chitosan, and dextran.
11. The composition according to item 10, wherein the water-soluble polymer is a cellulose derivative.
12. The composition according to item 11, wherein the cellulose derivative is hydroxypropylcellulose.
13. The composition according to item 1, wherein the composition is in the form of a tablet.
14. The composition according to item 1, wherein the composition is granulated.
15. The composition according to item 1, wherein the composition is in the form of nanoparticles having an effective average size of about 2000 nm or less.
16. The composition according to item 15, wherein the effective average size of the nanoparticles is about 1000 nm or less.
17. The composition according to item 16, wherein the effective average size of the nanoparticles is about 500 nm or less.
18. The composition according to item 1, comprising polycarbophil, hydroxypropyl cellulose, and an enzyme selected from the group consisting of lactase, lipase, and protease.
19. The composition according to item 18, wherein the enzyme is lactase.
20. A composition comprising:
   (a) at least one mucoadhesive polymer that is capable of forming a hydrogel;
   (b) at least one water-soluble polymer; and
   (c) at least one microorganism of a strain that produces a therapeutically effective amount of at least one enzyme.
21. The composition according to item 20, wherein the microorganism is selected from bacteria and fungi.
22. The composition according to item 21, wherein the microorganism is a bacteria.
23. The composition according to item 22, wherein the bacteria is selected from bifidobacteria and lactobacilli.
24. The composition according to item 23, wherein the bacteria is selected from the group consisting of *Bifidobacterium lactis, L. acidophilus, L. rhamnosus,* and *L. plantarum.*
25. The composition according to item 21, wherein the microorganism is a fungus.
26. The composition according to item 25, wherein the fungus is a yeast.
27. The composition according to item 20, wherein the mucoadhesive polymer is selected from the group consisting of polyacrylate and/or polymethacrylate polymers.
28. The composition according to item 20, wherein the water-soluble polymer is selected from the group consisting of cellulose, cellulose derivatives, chitosan, and dextran.
29. A composition comprising:
   (a) at least one mucoadhesive polymer that is capable of forming a hydrogel;
   (b) at least one water-soluble polymer; and
   (c) a therapeutically effective amount of at least one antibody.
30. The composition according to item 29, wherein the antibody is selected from the group consisting of antitumor necrosis factor alpha monoclonal antibodies, anti-interleukin-12 antibodies, and anti-rotaviral antibodies.
31. The composition according to item 30, wherein the antibody is an antitumor necrosis factor alpha monoclonal antibody.
32. The composition according to item 31, wherein the antitumor necrosis factor alpha monoclonal antibody is infliximab.
33. The composition according to item 29, wherein the mucoadhesive polymer is selected from the group consisting of polyacrylate and/or polymethacrylate polymers.
34. The composition according to item 33, wherein the polyacrylate-methacrylate polymers are selected from carbopols and anionic polymers of methacrylic acid esters.
35. The composition according to item 29, wherein the water-soluble polymer is selected from the group consisting of cellulose, cellulose derivatives, chitosan, and dextran.
36. The composition according to item 35, wherein the water-soluble polymer is a cellulose derivative.
37. The composition according to item 36, wherein the cellulose derivative is hydroxypropylcellulose.
38. A method of contacting at least one enzyme with a mucosal surface in a subject in need of the enzyme comprising administering to the subject a composition according to item 1.
39. The method according to item 38, wherein the administering occurs by oral administration of the composition.
40. The method according to item 38, wherein the mucosal surface is selected from the group consisting of the stomach, the small intestine, the large intestine, and the mouth.
41. The method according to item 40, wherein the mucosal surface is the small intestine.
42. The method according to item 40, wherein the mucosal surface is the mouth.
43. The method according to item 38, wherein the administering occurs by application of the composition to the mucosal surface.
44. The method according to item 38, wherein the mucosal surface is selected from the group consisting of corneal, conjunctival, nasal, buccal, sublingual, uteral, bladder, rectal, and vaginal mucosa surfaces.
45. The method of item 38, wherein the composition comprises nattokinase.
46. The method of item 38, wherein the composition comprises a digestive enzyme.
47. The method of item 38, wherein the composition comprises transglucosidase.
48. The method of item 38, wherein the composition comprises bilirubin oxidase.
49. A method of aiding digestion in a subject in need thereof, comprising administering to the subject at least one composition according to item 2.
50. A method of preventing the digestion of carbohydrates in a subject, comprising administering to the subject a composition according to item 2.
51. The method according to item 50, wherein the enzyme is selected from the group consisting of levansucrase, biodiastase, and a combination thereof.
52. A method of stimulating the growth of probiotic bacteria in a subject comprising administering to a subject a composition according to item 1.
53. A method of contacting at least one enzyme with a mucosal surface in a subject in need of the enzyme comprising administering to the subject a composition according to item 20, wherein the microorganism produces a therapeutically effective amount of the enzyme that is to be contacted with the mucosal surface.
54. A method of treating a subject suffering from a gastrointestinal or gastrointestinal-related disorder, said method comprising administering to the subject an effective amount of the composition according to item 29.
55. The method according to item 54, wherein the disorder is selected from the group consisting of Crohn's disease, ulcerative colitis, lymphomas, rejection after intestinal transplantation, and a viral gastroenteritis.
56. The method according to item 55, where the disorder is Crohn's disease.
57. The method according to item 55, where the disorder is a viral gastroenteritis.
58. The method according to item 57, wherein the viral gastroenteritis is rotovirus.
59. The method according to item 54, wherein subject is a human being.

## Claims

1. A composition comprising: a mixture of
(a) at least one mucoadhesive polymer that is capable of forming a hydrogel;
(b) at least one water-soluble polymer; and
(c)
(i) a therapeutically effective amount of at least one enzyme,
(ii) at least one microorganism of a strain that produces a therapeutically effective amount of at least one enzyme, or
(iii) a therapeutically effective amount of at least one antibody.
wherein the composition is suitable for oral administration and adheres to an intestinal mucosal surface.

2. The composition according to claim 1, wherein the composition comprises a digestive enzyme or a therapeutic enzyme.

3. The composition according to claim 2, wherein
(a) the digestive enzyme is selected from the group consisting of exoprotease, endoprotease, and acid stable proteases, bromelain, papain, and actinidin, endocellulase, glucanase, hemicellulase, cellulase, pectinase, lipase, lipase blends, alpha amylase, beta amylase, amyloglucosidase, lactase, invertase, maltase, xylanase, alpha galactosidase, mannanase, and combinations thereof; or
(b) the therapeutic enzyme is selected from the group consisting of transglucosidase, bilirubin oxidase, β-glycosidase, oxalate oxidase, phytase, collagenase, nattokinase, fructofuranosidase, levansucrase, and combinations thereof.

4. The composition according to claim 1, comprising polycarbophil, hydroxypropyl cellulose, and an enzyme selected from the group consisting of lactase, lipase, and protease.

5. The composition according to claim 1, wherein the microorganism is selected from bacteria and fungi.

6. The composition according to any one of the preceding claims, wherein
(a) the mucoadhesive polymer is selected from the group consisting of polyacrylate and/or polymethacrylate polymers; and/or
(b) the water-soluble polymer is selected from the group consisting of cellulose, cellulose derivatives, chitosan, and dextran.

7. The composition according to any one of the preceding claims, wherein
(a) the composition is in the form of a tablet;
(b) the composition is granulated; or
(c) the composition is in the form of nanoparticles having an effective average size of about 2000 nm or less, about 1000 nm or less, or about 500 nm or less.

8. The composition of any one of the preceding claims for use in therapy, wherein at least one enzyme or antibody contacts a mucosal surface.

9. The composition according to claim 8, wherein
(a) the composition is to be administered orally or is to be administered via application to the mucosal surface; and/or
(b) the composition comprises nattokinase, transglucosidase, or bilirubin oxidase.

10. The composition according to claim 8, wherein the mucosal surface is selected from the group consisting of the stomach, the small intestine, and the large intestine.

11. The composition according to claim 2 for use in aiding digestion or for preventing the digestion of carbohydrates in a subject.

12. The composition according to claim 11, wherein the enzyme for preventing the digestion of carbohydrates is selected from the group consisting of levansucrase, biodiastase, and a combination thereof.

13. The composition according to claim 8 for use in stimulating the growth of probiotic bacteria in a subject.

14. The composition according to claim 8 for use in treating a gastrointestinal or gastrointestinal-related disorder in a subject.

15. The composition according to claim 14, wherein the disorder is selected from the group consisting of Crohn's disease, ulcerative colitis, lymphomas, rejection after intestinal transplantation, and a viral gastroenteritis.
